(19) Europäisches Patentamt
European Patent Office
Office européen des brevets

(11) Veröffentlichungsnummer: **0 002 096**
**B1**

(12) # EUROPÄISCHE PATENTSCHRIFT

(45) Veröffentlichungstag der Patentschrift: **30.09.81**

(21) Anmeldenummer: **78200311.5**

(22) Anmeldetag: **20.11.78**

(51) Int. Cl.³: **C 07 D 239/06,**
C 08 G 18/80

(54) **Verfahren zur Herstellung von blockierten Polyisocyanaten und danach erhältliche blockierte Polyisocyanate.**

(30) Priorität: **19.11.77 DE 2751805**

(43) Veröffentlichungstag der Anmeldung:
**30.05.79 Patentblatt 79/11**

(45) Bekanntmachung des Hinweises auf die Patenterteilung:
**30.09.81 Patentblatt 81/39**

(84) Benannte Vertragsstaaten:
**BE CH DE FR GB LU NL**

(56) Entgegenhaltungen:
**DE - A - 2 156 881**

(73) Patentinhaber: **CHEMISCHE WERKE HÜLS AG**
**Postfach 1320**
**D-4370 Marl 1 (DE)**

(72) Erfinder: **Schott, Ansgar, Dr.**
**Steinring 70**
**D-4630 Bochum (DE)**
Erfinder: **Gras, Rainer, Dr.**
**An der Ziegelei 91**
**D-4690 Herne 2 (DE)**
Erfinder: **Wolf, Elmar, Dr.**
**Am Böckenbusch 3a**
**D-4690 Herne 2 (DE)**

(74) Vertreter: **Steil, Hanna, Dipl.-Chem., et al**
**RSP PATENTE-PB 40**
**Holsterhauser Strasse 160**
**Postfach 2840**
**D-4690 Herne 2 (DE)**

**EP 0 002 096 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von blockierten Polyisocyanaten und danach erhältliche blockierte Polyisocyanate

Gegenstand der vorliegenden Erfindung sind ein Verfahren zur Herstellung von neuen Verbindungen mit hohem latenten Isocyanatgehalt sowie die nach diesem Verfahren erhältlichen Verbindungen.

Die Herstellung von verkappten Isocyanaten, auch Isocyanatabspalter genannt, ist bekannt und wird im Houben-Weyl, Methoden der organischen Chemie XIV/2 S. 61—70, beschrieben. Als Blockierungsmittel sind tertiäre Alkohole, Phenole, Mercaptane, Acetessigester, Malonsäureester, Acetylaceton, Imide, wie Phthalimid, Imidazole, Chlorwasserstoff, Cyanwasserstoff und ε-Caprolactam bekannt. In der Praxis ist die Verwendung von Monophenolen und ε-Caprolactam für diesen Einsatzzweck bevorzugt worden.

Diese verkappten Isocyanate besitzen die Eigenschaft, bei erhöhter Temperatur wie Isocyanate zu reagieren. Die Abspaltung des Blockierungsmittels erfolgt umso leichter, je saurer das H-Atom der Maskierungsgruppe ist. Derartige blockierte Isocyanate werden in der DT-OS 21 66 423 beschrieben. Auch sind endständige blockierte Isocyanate, die zusätzlich noch Uretdiongruppen enthalten, in der DT-OS 25 02 934 beschrieben worden.

Der bedeutendste Nachteil bei der Verwendung dieser Blockierungsmittel ist bei einer Reihe von Einsatzzwecken die relativ hohe Abspalttemperatur, die für die meisten Phenole bei aliphatischen Polyisocyanaten bei mindestens 190°C und bei aromatischen Polyisocyanaten ca. 30°C niedriger liegt. Die Aufspalttemperatur von aliphatischen ε-Caprolactamblockierten Polyisocyanaten liegt beispielsweise im allgemeinen bei ca. 180—190°C.

Aus diesem Grunde ist in der Vergangenheit vorgeschlagen worden, Thiophenole zu verwenden, für die diese Temperatur viel niedriger ist. Der Nachteil der Thiophenole besteht jedoch in ihrem außergewöhnlich unangenehmen Geruch.

Es wurde auch vorgeschlagen, 1—2 Prozent eines Katalysators hinzuzufügen, mittels welcher die Abspalttemperatur bis auf 125—140°C reduziert werden könnte. Auf diese Weise wird jedoch die Gebrauchsdauer des ungehärteten Beschichtungsmaterials ungünstig beeinflußt.

Überraschenderweise wurde nunmehr gefunden, daß es durchaus möglich ist, auf einfache Weise blockierte Polyisocyanate herzustellen, die bei einer um mindestens 20—30°C niedrigeren Temperatur als die mit den herkömmlichen Blockierungsmitteln verkappten Isocyanaten deblockieren, wenn man das Verfahren zur Herstellung von blockierten Polyisocyanaten durch Umsetzung von Polyisocyanaten mit N-haltigen cyclischen Verbindungen so durchführt, daß man als N-haltige cyclische Verbindung cyclische Amidine der allgemeinen Formel

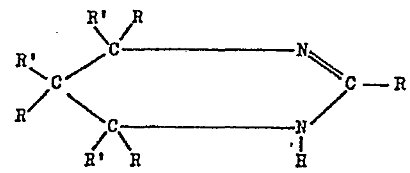

einsetzt, worin R gleiche oder verschiedene Substituenten aus de Gruppe Wasserstoff, Alkyl-, Cycloalkyl-, Aralkyl- und Arylrest, sowie R′ R oder 2 R′ gemeinsam Bestandteil eines cycloaliphatischen Ringes sein können und die Umsetzung bei Temperaturen von 0—150°C, vorzugsweise von 80 bis 120°C erfolgt.

Die Polyisocyanate und die cyclischen Amidine werden in solchen Mengen eingesetzt, daß auf eine Isocyanatgruppe 0,5—1,1, vorzugsweise 0,8—1,0 Mol, cyclisches Amidin kommen.

Als Ausgansverbindungen, die zur Blockierung mit den cyclischen Amidinen eingesetzt werden können, eignen sich beispielsweise Polyisocyanate, insbesondere Diisocyanate, wie aliphatische, cycloaliphtische, araliphatische, d.h. arylsubstituierte aliphatische und/oder aromatische Diisocyanate, wie sie beispielsweise in Houben-Weyl, Methoden der organischen Chemie, Band 14/2, S. 61—70 und dem Artikel von W. Siefken in Justus Liebigs Annalen der Chemie *562*, 75 bis 136, beschrieben werden, wie 1,2-Äthylendiisocyanat, 1,4 - Tetramethylendiisocyanat, 1,6 - Hexamethylendiisocyanat, 2,2,4 - bzw. 2,4,4 - Trimethyl - 1,6 - hexamethylendiisocyanat (TMDI), 1,12 - Dodecandiisocyanat, ω,ω′ - Diisocyanatodipropyläther, Cyclobutan - 1,3 - diisocyanat, Cyclohexan - 1,3 - und 1,4 - diisocyanat, 2,2- und 2,6 - Diisocyanato - 1 - methylcyclohexan, 3 - Isocyanatomethyl - 3,5,5 - trimethylcyclohexylisocyanat, welches auch als Isophorondiisocyanat bezeichnet und mit IPDI abgekürzt wird, Decahydro - 8 - methyl - (1,4 - methano - naphthalen - 2(oder 3)) - 5 - ylendimethylen-diisocyanat, Hexahydro - 4,7 - methano-indan - 1 (oder 2) - 5 - (oder 6) - ylendimethylen-diisocyanat, Hexahydro - 4,7 - methanindan, - 1- (oder 2) - 5 - (oder 6) - ylen/diisocyanat, Dicyclohexyl - 4,4′ - diisocyanat, Dicyclohexyl - 2,4′ - diisocyanat, 2,4 - und 2,6 - Hexahydrotoluylendiisocyanat, Perhydro - 2,4′ - und/oder - 4,4′ - diphenylmethan-diisocyanat, ω,ω′, - Diisocyanato - 1,4 - diäthylbenzol, 1,3 - und 1,4 - Phenylendiisocyanat, 4,4′ - Diisocyanato-diphenyl, 4,4′ - Diisocyanato - 3,3′ - dichlordiphenyl, 4,4′ - Diisocyanato - 3,3′ - dimethoxy-diphenyl - 4,4′ - Diisocyanato - 3,3′ - dimethyl-diphenyl, 4,4′ -

Diisocyanato - 3,3' - diphenyl - diphenyl, 2,4' - und 4,4' - Diisocyanato-diphenylmethan, Naphthylen - 1,5 - diisocyanat,Toluylendiisocyanate, 2,4 - bzw. 2,6 - Toluylen-diisocyanat, N,N' - (4,4' - Dimethyl - 3,3' - diisocyanatodiphenyl) - uretdion, m - Xylylen-diisocyanat, aber auch die Triisocyanate wie 2,4,4' - Triisocyanato-diphenyläther, 4,4',4'' - Triisocyanatotriphenylmethan, Tris - (4 - isocyanatophenyl) - thiophosphat, sowie beliebige Gemische dieser Isomeren. Weitere geeignete Isocyanate werden in dem genannten Artikel in den Annalen auf Seite 122 f. beschrieben.

Besonders bevorzugt werden in der Regel die technisch leicht zugängigen aliphatischen, cycloaliphatischen oder aromatischen Diisocyanate und besonders das 3 - Isocyanatomethyl - 3,5,5 - trimethyl - cyclohexylisocyanat und 2,4 - bzw. 2,6 - Toluylendiisocyanat sowie deren isomere Gemische.

Neben den monomeren Polyisocyanaten können als Ausgangsstoffe für die Blockierung mit den nachstehend ausführlich beschriebenen Tetrahydropyrimidinen selbstverständlich auch die dimeren und trimeren Formen der Polyisocyanate, wie Uretdionen und Isocyanurate, eingesetzt werden, die nach bekannten Methoden herstellbar sind.

Unter Polyisocyanaten im Sinne der vorliegenden Erfindung werden auch solche verstanden, die vor der Blockierung mit den Tetrahydropyrimidinen einer Umsetzung zur Molekülvergrößerung mit den in der Isocyanatchemie gebräuchlichen sogenannten Kettenverlängerungsmitteln, wie Wasser, Polyolen, Polyaminen u.a., unterworfen wurden, wobei das bi- oder trifunktionelle Kettenverlängerungsmittel, also solche mit 2 oder 3 gegenüber Isocyanatgruppen reaktionsfähigen Gruppen, wie Hydroxyl-und/oder Aminogruppen, tragende Verbindungen, in solchen Mengen verwendet wird, daß das resultierende neue Isocyanat im Durchschnitt mindestens 2 Isocyanatgruppen trägt. Bei Verwendung von Wasser als Kettenverlängerungsmittel resultieren Polyisocyanate mit einer oder mehreren Harnstoffgruppierungen. Die Molekulargewichte der geeigneten Kettenverlängerungsmittel liegen zweckmäßigerweise zwischen 18 und 250.

Geeignete Polyole sind beispielsweise Diole und Triole, z.B. Äthylenglykol, Propylenglykole, wie 1,2 - und 1,3 - Propandiol, 2,2 - Dimethylpropandiol - (1,3), Butandiole, wie Butandiol - (1,4), Hexandiole, z.B. Hexandiol - (1,6),2,2,4 - Trimethylhexandiol - (1,6), 2,4,4 - Trimethylhexandiol - (1,6), Heptandiol - (1,7), Octadecen - 9,10 - diol - (1,12), Thiodiglykol, Octadecandiol - (1,18), 2,4 - Dimethyl - 2 - propylheptandiol - (1,3), Buten- oder Butindiol - (1,4), Diäthylenglykol, Triäthylenglykol, Tetraäthylenglykol, trans-und cis - 1,4 - Cyclohexandimethanol,1,4 - Cyclohexandiole, Glycerin, Hexantriol - (1,2,6), 1,1,1 - Trimethylolpropan, 1,1,1 - Trimethyloläthan u.a. Es können auch Mischungen der vorgenannten Verbindungen verwendet werden.

Von den für die Kettenverlängerung bzw. Molekülvergrößerung geeigneten Polyaminen sollen beispielsweise das Äthylendiamin - 1,2, Propylendiamin - 1,2 und - 1,3, Butylendiamin - 1,2, - 1,3 und - 1,4 sowie die Hexamethylendiamine, die auch einen oder mehrere $C_1$—$C_4$ Alkylrest tragen können.

Die Umsetzung der Polyisocyanate vor der Blockierung mit den genannten Kettenverlängerungsmitteln in den angegebenen Mengenverhältnissen kann bei Temperaturen im Bereich von 0—150°C, vorzugsweise 80—120°C durchgeführt werden.

Geeignete Tetrahydropyrimidin-Derivate im Sinne der vorliegenden Erfindung, die der früher beschriebenen allgemeinen Formel entsprechen, sind beispielsweise solche mit gegebenenfalls arylsubstituierten Alkylresten, mit gegebenenfalls alkylsubstituierten Arylresten, wie 2 - Methyltetrahydropyrimidin, 2,4 - (5 oder 6), Dimethyltetrahydropyrimidin, 2 - Äthyltetrahydropyrimidin, 2 - Äthyl - 4 - methyl - tetrahydropyrimidin, 2 - Benzyltetrahydropyrimidin, 2 - Phenyl-tetrahydropyrimidin, 2 - Phenyl - 4 (5 oder 6) - methyl - tetrahydropyrimidin, 2,4 - Diaza - 3 - phenyl - 7,9,9 - (oder 7,7,9) - trimethyl - bicyclo - (4.3.0) - nonen - (2), 2,4 - Diaza - 3 - methyl - 7,9,9 - (oder 7,7,9) - trimethyl - bicyclo - [4,3,0] - nonen - (2) u.a.m. Es können auch Gemische der Tetrahydropyrimidin-Derivate erfindungsgemäß eingesetzt werden. Dieses ist dann besonders zweckmäßig, wenn blockierte Isocyanate mit niedrigen Schmelzpunkten bzw. - bereichen benötigt werden.

Die erfindungsgemäß einsetzbaren Tetrahydropyrimidin-Derivate können nach bekannten Verfahren aus gegebenenfalls substituierten 1,3 - Diaminen und aliphatischen oder aromatischen Monocarbonsäuren, Mononitrilen oder Monocarbonsäureestern, gegebenenfalls in Gegenwart von elementarem Schwefel oder Sulfurylchlorid als Katalysator hergestellt werden.

Die DE - A - 2 156 881 betrifft Epoxyisocyanate. Es werden dort Epichlorohydrin mit Hydtantoin und einem Polyisocyanat umgesetzt. Während cyclische Amidine besischen Stickstoff und eine —C=N— Doppelbindung aufweisen, besitzen die Hydantoine keimen besischen Stickstoff und keine —C=N— Doppelbindung. Unter Härtungsbedingungen triff bei mit cyclischen Amidinen blockierten Polyisocyanaten eine Rückspaltung auf, während dies bei den Verbindungen gemäß DE—AJ 2 156 881 nicht der Fall ist, Letztere werden mit Aminen vernetzt.

Die Blockierung kann, wie bereits erwähnt, auch in Lösungsmitteln durchgeführt werden. Als Lösungsmittel für diese Reaktion kommen

nur solche infrage, die mit den Polyisocyanaten nicht reagieren, beispielsweise Ketone, wie Aceton, Methyläthylketon, Methylisobutylketon, Cyclopentanon, Cyclohexanon u.a.; Aromaten, wie Benzol, Toluol, Xylole, Chlorbenzol, Nitrobenzol u.a.; cyclische Äther, wie Tetrahydrofuran, Dioxan u.a.; Ester, wie Methylacetat, n-Butylacetat u.a. aliphatische Chlorkohlenwasserstoffe, wie Chloroform, Tetrachlorkohlenstoff u.a.; sowie aprotische Lösungsmittel, wie Dimethylformamid, Dimethylacetamid, Dimethylsulfoxid usw.

Wenn das Blockierungsmittel im Verhältnis von $\geqq 1$ zur Anzahl der Isocyanatgruppen eingesetzt wird, werden, die Reaktionsmischungen so lange bei den angegebenen Temperaturen gehalten, bis der NCO-Gehalt der Reaktionsmischung auf Werte unter 0,2% NCO abgesunken ist, für das Verhältnis <1 bis zur Erreichung eines konstanten NCO-Wertes.

Ein weiterer Gegenstand der vorliegenden Erfindung ist noch die nachträgliche Umsetzung einer Gruppe von blockierten Polyisocyanaten, nämlich solche, bei denen die cyclischen Amidine in unterstöchiometrischen Mengen eingesetzt worden sind, d.h. das Verhältnis cyclisches Amidin zu Isocyanatgruppen war <1:1 mit den gleichen Kettenverlängerungsmitteln, die bereits früher als Mittel zur Molekülvergrößerung beschrieben worden sind. Die Umsetzung erfolgt ebenfalls bei Temperaturen im Bereich von 0—150°C, vorzugsweise 80—120°C, jedoch unterhalb der Deblockierungstemperatur des blockierten Polyisocyanats wobei gegebenenfalls überschüssigen Kettenverlängerungsmittel alle in oder mit gegebenenfalls eingesetzten Lösungsmittel auf bekonnte Weise entferntwird. Durch diese Verfahrensvariante läßt sich die Produktpalette der blockierten Polyisocyanate in sehr weiten Grenzen den praktischen Erfordernissen anpassen. Diese Verfahrensvariante ist besonders von Interesse für Polyisocyanate mit unterschiedlich reaktiven NCO-Gruppen, d.h. asymmetrischen Polyisocyanaten.

So lassen sich durch Wechsel der Reihenfolge Adduktbildung/Blockierung blockierte Polyisocyanate mit unterschiedlicher Reaktivität, Schmelzbereich und Struktur erhalten.

Gegenstand der Erfindung sind weiter auch die nach den beschriebenen Verfahren erhältlichen Addukte, deren allgemeinen Formeln in den Ansprüchen niedergelegt wurden. Es handelt sich dabei im allgemeinen um Verbindungen des Molekulargewichtsbereichs von 250—3 000, vorzugsweise von 300—1000. Die Verfahrensprodukte besitzen einen Schmelzbereich im Temperaturbereich von 30—220°C, vorzugsweise 80—160°C. Die mit den cyclischen Amidinen blockierten Polyisocyanate sind weiter durch einen Gehalt an endständig in blockierter Form vorliegenden Isocyanatgruppen (berechnet als NCO) von 4—26 Gew.%, vorzugsweise 7—23 Gew.%, charakterisiert.

Die Verbindungen der vorstehend beschriebenen Erfindung eignen sich besonders wegen ihrer Schmelzpunkte bei gleichzeitig höheren Molekulargewichten als Katalysatoren für die anionische Polymerisation von ε-Caprolactam. Die Verbindungen können auch zur Herstellung von Drahtlacken verwendet werden.

Das Verfahren und die resultierenden Verbindungen werden durch die nachstehenden Beispiele illustriert:

Beispiel 1

1 Herstellung des Tetrahydropyrimidin-Isomerengemisches

312 g eines Isomerengemisches aus 1 - Amino - 2 - aminomethyl - 3.3.5 bzw. 3.5.5 - trimethylcyclopentan (TMCPD) wurden mit 136 g Benzoësäuremethylester vermischt und unter Rührung in einem Reaktor auf 190°C aufgeheizt. Die Reaktionsdauer betrug 2 Stunden. Nach dieser Zeit wurde der Diaminüberschuß zusammen mit den Abspaltprodukten Methanol und Wasser abdestilliert. Im Vakuum bei 0,1 Torr ließ sich in einem Temperaturbereich von 158—170°C ein Isomerengemisch von bicyclischen Tetrahydropyrimidinen in einer Ausbeute von 208 g ≙ 86% destillieren.

Das zähflüssige, gelbe Produkt ist ein Gemisch von Verbindungen der nachstehenden Formeln

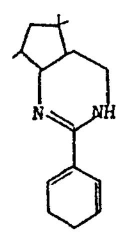
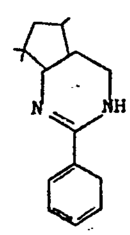

DieC/H/N-Analyse ergab folgende Werte:
79,13% C, 9,3% H, 11,75% N,
(Theorie: 79,34% C, 9,09% H, 11,57% N)
$NH_2$-Gehalt: 4,11 mMol $NH_2$/g (theor. 4,13)
MG: 243,3 (theor. 242)

2 Isocyanatblockierungen

2a 484 g des bicyclischen Isomerengemisches entsprechend Beispiel 1 wurden in einem Kolben auf 100°C erhitzt. Innerhalb von 90 Minuten wurden 222 g 3,3,5 - Trimethyl - 1 - isocyanato - 5 - isocyanatomethylcyclohexen bzw. 3,5,5 - Trimethyl - 1 - isocyanato - 3 - isocyanatomethylcyclohexan (IPDI) (1:1) zugetropft. Während dieser Zeit erhöhte sich die Viskosität sehr stark. Zur Vervollständigung der Reaktion wurde der Ansatz noch 180 Minuten auf 130°C erhitzt.

Der Glasumwandlungspunkt (DTA) des resultierenden Gemisches lag bei 67—75°C, der Schmelzbereich bei 85—98°C und die Aufspalttemperatur bei ca. 160°C. Das IR-Spektrum des blockierten Polyisocyanates zeigt Abb. 1.

2b Analog Beispiel 2a wurde aus 210 g 2,2,4 - bzw. 2,4,4 - Trimethylhexamethylen - diisocyanat (TMDI) (1:1), 484 g des Gemisches aus Beispiel 1, bei 100°C das entsprechende Isocyanataddukt hergestellt. Schmelzbereich: 75—89°C, Erweichungspunkt (DTA): 52—75°C, Aufspalttemperatur: 160°C. Das IR-Spektrum des blockierten Polyisocyanates zeigt Abb. 2.

2c Analog Beispiel 2a wurde aus 175 g Toluylen - 2,4 - (2,6) - diisocyanat (TDI) (aus 80% 2,4 und 20% 2,6) und 484 g des Gemisches aus Beispiel 1 bei 120°C das entsprechende Isocyanataddukt hergestellt; gelbes Pulver. Schmelzbereich 93—105°C, Erweichungspunkt (DTA) 71—89°C, Aufspalttemperatur 150°C. Das IR-Spektrum des erhaltenen blockierten Polyisocyanates zeigt Abb. 3.

Beispiel 3

Herstellung der Diäthylenglykol/Isocyanat - Addukte

3a) Zu 444 g IPDI entsprechend Beispiel 2a wurden bei 80°C unter guter Rührung 106 g Diäthylenglykol langsam zugegeben. Nach erfolgter Diäthylenglykolzugabe wurde noch 2 h bei 80°C weiter erhitzt. Der NCO-Gehalt des Adduktes betrug dann 15,1 Gew.%.

3b) Zu 420 g TMDI entsprechend Beispiel 2b wurden bei 90°C unter guter Rührung 106 g Diäthylenglykol langsam zugegeben. Nach erfolgter Diäthylenglykolzugabe wurde noch 2 h bei 90°C weiter erhitzt. Der NCO-Gehalt des Adduktes betrug dann 16,0 Gew.%.

3c) Zu 348 g TDI entsprechend Beispiel 2c wurden bei 95°C unter guter Rührung 106 g Diäthylenglykol langsam zugegeben. Nach erfolgter Diäthylenglykolzugabe wurde noch 2 h bei 90°C weiter erhitzt. Der NCO-Gehalt des Adduktes betrug dann 18,5 Gew.%.

Beispiel 4

Blockierte Isocyanat-Addukte

4a Zu 550 g des im Beispiel 3a beschriebenen Adduktes aus 2 Molen IPDI und 1 Mol Diäthylenglykol wurden bei 120°C 484 g des Gemisches entsprechend Beispiel 1 so zugegeben, daß die Temperatur nicht über 125°C anstieg. Nach erfolgter Zugabe wurde das Reaktionsgemisch noch weitere 2 h bei 120°C erhitzt.

In dem so hergestellten Reaktionsprodukt konnte kein NCO mehr nachgewiesen werden. Das Reaktionsprodukt stellt ein gelbliches Pulver mit einem Schmelzbereich von 89—101°C und einem Erweichungspunkt (DTA) von 75—86°C mit Aufspalttemperatur 170°C dar. Das IR-Spektrum des erhaltenen blockierten Polyisocyanatadduktes zeigt Abb. 4.

4b Analog Beispiel 4a wurden 526 g des im Beispiel 3b beschrieben Adduktes aus 2 Molen TMDI und 1 Mol Diäthylenglykol mit 484 g des Gemisches entsprechend Beispiel 1 bei 120°C umgesetzt. Das Reaktionsprodukt ist spröde und gelb gefärbt. Schmelzbereich 77—93°C, Erweichungspunkt (DTA) 69—79°C, Aufspalttemperatur 170°C.

4c Analog Beispiel 4a wurden 454 g des im Beispiel 3c beschriebenen Adduktes mit 484 g des Gemisches entsprechend Beispiel 1 bei 135°C umgesetzt. Schmelzbereich 93—107°C, Erweichungspunkt (DTA) 79—92°C, Aufspalttemperatur 160°C.

Beispiel 5

Herstellung eines weiteren Tetrahydropyrimidingemisches

216 g Essigsäureäthylester und 1248 g TMCPD-Isomerengemisch (Molverhältnis 1:4) wurden in einen Druckreaktor mit Rührung eingefüllt und 2,5 h bei 190°C und 6,5 bar zur Reaktion gebracht. Nach Entfernen des überschüssigen TMCPD und der Abspaltprodukte duch Destillation ließ sich im Vakuum (0,5 Torr) bei 100—119°C ein Isomerengemisch mit Tetrahydropyrimidinstruktur isolieren. Ausbeute: 274 g ≙ 76% d. Th..

Das hochviskose, zähflüssige Produkt ist gelblich gefärbt und enthält Verbindungen folgender Formeln

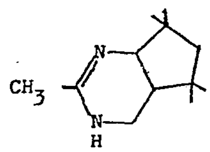

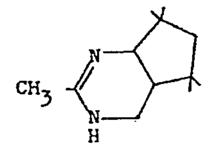

Äquivalentgewicht: 183, Theorie: 180
Gefunden: 73,12% C, 10,89% H, 15,45% N
Theorie: 73,33% C, 11,11% H, 15,55% N

Beispiel 6

Isocyanatblockierung

6a) Zu 360 g des Gemisches entsprechend Beispiel 5 wurden 222 g IPDI entsprechend Beispiel 2a so zugetropft, daß die Temperatur im Reaktionskolben nicht über 120°C stieg. Zur Vervollständigung der Reaktion wurde die Reaktionsmischung noch 3 h bei 120°C gehalten. Diese Bedingungen reichten für eine nahezu vollständige Umsetzung aus. Das Reaktionsprodukt ist ein gelbliches kristallines Pulver mit einem Schmelzbereich von 95 bis 104°C, einem Erweichungspunkt (DTA) von 59—74°C und einem freien NCO-Gehalt von 0,1%, Aufspalttemperatur lag bei 180°C. Das IR-Spektrum des blockierten Polyisocyanates zeigt Abb. 5.

6b) Zu 360 g des Gemisches entsprechend Beispiel 5 wurden 210 g TMDI entsprechend Beispiel 2b so zugetropft, daß die Temperatur im Reaktionskolben 120°C nicht überstieg. Zur Vervollständigung der Reaktion wurde die Reaktionsmischung noch 3 h bei 120°C gehalten. Die Bedingungen reichten für eine vollständige Umsetzung aus. Das Reaktionsprodukt hatte einen Schmelzbereich von 84—95°C, einen Erweichungspunkt (DTA) von 50—69°C und einen freien NCO-Gehalt von 0,1%. Die Aufspalttemperatur lag bei ca. 170°C. Das IR-Spektrum des blockierten Polyisocyanates zeigt Abb. 6.

6c) Analog Beispiel 6a wurde aus 360 g des Gemisches entsprechend Beispiel 5 und 174 g entsprechend Beispiel 2c bei 125°C das entsprechende Isocyanatprodukt hergestellt: gelbes Reaktionsprodukt, Schmelzbereich: 98—111°C, Erweichungspunkt (DTA) 70—91°C, Aufspalttemperatur 170°C. Das IR-Spektrum des blockierten Polyisocyanates zeigt Abb. 7.

Beispiel 7

Blockierte Isocyanat-Addukte

7a) Zu 550 g des im Beispiel 3a beschriebenen Addukts aus 2 Molen IPDI und 1 Mol Diäthylenglykol wurden 360 g des Gemisches entsprechend Beispiel 5 so zugetropft, daß die Temperatur nicht über 130°C anstieg. Nach erfolgter Zugabe wurde das Reaktionsgemisch noch weitere 2 h bei 130°C gehalten. In dem so hergestellten Reaktionsprodukt konnte kein NCO mehr nachgewiesen werden. Das Reaktionsprodukt ist ein fabloses Pulver mit einem Schmelzbereich von 103—113°C, einem Erweichungspunkt von 77—92°C und einer Aufspalttemperatur von 190°C. Das IR-Spektrum des blockierten Polyisocyanates zeigt Abb. 8.

7b) Zu 526 g des in Beispiel 3b beschriebenen Adduktes wurden 360 g des Gemisches entsprechend Beispiel 5 so zugegeben, daß die Temperatur nicht über 130°C anstieg. Nach erfolgter Zugabe wurde das Reaktion gemisch noch weitere 2 h bei 130°C gehalten. Das Reaktionsprodukt ist ein gelbliches, NCO-freies Pulver mit einem Schmelzbereich von 98—106°C, einem Erweichungspunkt (DTA) von 69—88°C. Die Aufspalttemperatur lag bei 180°C.

7c) Analog Beispiel 7a wurden 454 g des im Beispiel 3c beschriebenen Adduktes aus 2 Mol TDI und 1 Mol Diäthylenglykol mit 360 g des Gemisches entsprechend Beispiel 5 bei 140°C umgesetzt. Das Reaktionsprodukt ist gelb gefärbt. Schmelzbereich 112—120°C, Erweichungspukt (DTA) 89—101°C, Aufspalttemperatur 170°C.

Beispiel 8

Herstellung von 2-Phenyl-tetrahydropyrimidin

185 g 1,3-Diaminopropan wurden 136 g Benzoesäuremethylester unter Druck bei 200°C im Reaktor zur Reaktion gebracht. Nach 3 h wurde der Überschuß Diamin abdestilliert und das 2 - Phenyl - tetrahydropyrimidin in 79%iger Ausbeute isoliert. Kp 165°C/1 mm; Fp=86—87°C.

Beispiel 9

Isocyanatblockierung

9a) Zu 338 g 2 - Phenyltetrahydropyrimidin wurden 222 g IPDI entsprechend Beispiel 2a so zugetropft, daß die Temperatur im Reaktionskolben nicht über 120°C stieg. Die Mischung wurde noch 3 h bei 120°C gehalten. Der Schmelzbereich des gelben Pulvers lag bei 71—85°C, Erweichungspunkt (DTA) 48—65°C, Aufspalttemperatur 160°C, NCO-Gehalt 0,1%. Das IR-Spektrum des blockierten Polyisocyanates zeigt Abb. 9.

9b) Eine 50%ige acetonische Lösung von 174 Gew.-Teilen Toluylendiisocyanat (TDI) entsprechend Beispiel 2c wurde zu einer 50%igen acetonischen Lösung von 338 g 2 - Phenyl - tetrahydropyrimidin in der Siedehitze zugetropft und 2 h unter Rückfluß erhitzt. Nach Abdestillieren des Acetons erhielt man ein hellgelbes Pulver. Schmelzbereich: 68 bis 91°C, Erweichungspunkt (DTA) 52—68°C, Aufspalttemperatur: 160°C, NCO-Gehalt <0,2%. Das IR-Spektrum des blockierten Polyisocyanates zeigt Abb. 10.

Beispiel 10

Herstellung des 2-Phenyl-4 bzw. 6-methyltetrahydropyrimidin-Isomeren-Gemisches

52,8 g 1,3 - Diaminobutan wurden mit 27,2 g Benzoesäuremethylester unter Druck bei 200°C im Reaktor zur Reaktion gebracht. Nach 3 h wurde der Überschuß Diamin abdestilliert und das 2 - Phenyl - 4 - bzw. 6 - methyltetrahydropyrimidin in 68%iger Ausbeute isoliert. Kp $_{0,05}$ 198°C, Äquivalentgewicht 182 (Theorie 174).

Beispiel 11

Isocyanatblockierung

Zu 36,6 g des Isomerengemisches gemäß Beispiel 10 wurden 22,2 g IPDI entsprechend Beispiel 2a bei 120°C zugetropft und 3 h bei dieser Temperatur gehalten. Der Schmelzbereich des gelben Produktes lag bei 70—83°C, Erweichungspunkt (DTA) 43 bis 58°C, Aufspalttemperatur 180°C, NCO-Gehalt <0,2%.

**Patentansprüche**

1. Verfahren zur Herstellung von blockierten Polyisocyanaten durch Umsetzung von Polyisocyanaten mit N-haltigen cyclischen Verbindungen, dadurch gekennzeichnet, daß man als N-haltige cyclische Verbindungen cyclische Amidine der allgemeinen Formel,

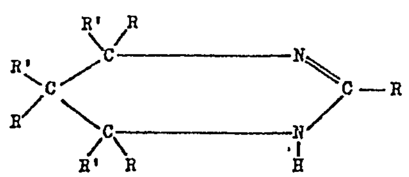

einsetzt, worin R gleiche oder verschiedene Substituenten aus der Gruppe Wasserstoff, Alkyl-, Cycloalkyl-, Aralkyl-und Arylrest sowie R′ R oder 2 R′ gemeinsam Bestandteil eines cycloaliphatischen Ringes sind und die Umsetzung bei Temperaturen von 0—150°C, vorzugsweise 80—120°C durchführt.

2. Verfahren nach Anspruch 1, dadurch

gekennzeichnet, daß man die Polyisocyanate und die cyclischen Amidine in solchen Mengen einsetzt, daß auf eine Isocyanatgruppe 0,5—1,1, vorzugsweise 0,8—1,0 Mol cyclisches Amidin kommen.

3. Verfahren nach Anspruch 2, dadurch gekennzeichnet, daß man die blockierten Polyisocyanate, bei denen cyclische Amidine in unterstöchiometrischen Mengen eingesetzt wurden, anschließend einer Umsetzung mit Kettenverlängerungsmitteln bei Temperaturen im Bereich von 0—150°C, jedoch unter der Deblockierungstemperatur, umsetzt und gegebenenfalls das überschüssige Kettenverlängerungsmittel entweder allein oder mit einem gegebenenfalls eingesetzten Lösungsmittel auf bekannte Weise entfernt.

4. Verfahren nach Anspruch 3, dadurch gekennzeichnet, daß man die Umsetzung bei Temperaturen im Bereich von 80—120°C durchführt.

5. Verfahren nach den Ansprüchen 3 und 4, dadurch gekennzeichnet, daß das Kettenverlängerungsmittel Wasser ist.

6. Verfahren nach den Ansprüchen 3 und 4, dadurch gekennzeichnet, daß die Kettenverlängerungsmittel Polyole sind.

7. Verfahren nach den Ansprüchen 3 und 4, dadurch gekennzeichnet, daß die Kettenverlängerungsmittel Polyamine sind.

**Revendications**

1. Procédé de fabrication de polyisocyanates bloqués formés par réaction de polyisocyanates avec des composés cycliques ayant azote caractérisé en ce qu'on utilise comme composés cycliques ayant azote les amidines cycliques de formule générale

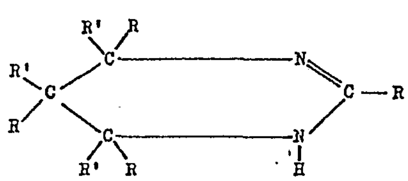

où R sont les mêmes ou différents substituents du groupe hydrogène et un reste alcoylène cycloalcoylène, aralcolyène et arylène et R'R ou 2 R' ensemble sont les parties d'anneau cycloaliphatique, et qu'on exécute la réaction à des températures entre 0 et 150°C, de préférence entre 80 et 120°C.

2. Procédé selon la revendication 1, caractérisé en ce qu'on utilise les polyisocyanates et les amidines cycliques en quantités telles que pour 1 groupe d'isocyanate sont utilisés 0,5—1,1, de préférence 0,8—1,0 mols, d'amidine cyclique.

3. Procédé selon la revendication 2, caractérisé en ce qu'on fait réagir les polyisocyanates bloqués, dans lesquelles on utilise moins que les quantités équivalents d'amidines cycliques, après cela avec des agents d'allongement de chaîne à des températures entre 0 et 150°C, cependant sous la température de la débloquage et le cas échéant enlève selon les méthodes connues seulement l'agent d'allongement de chaîne en excès ou de même le solvant appliqué le cas échéant.

4. Procédé selon la revendication 3, caractérisé en ce qu'on exécute la réaction à des températures entre 80 et 120°C.

5. Procédé selon les revendications 3 et 4, caractérisé en ce que l'agent d'allongement de chaîne et l'eau.

6. Procédé selon les revendications 3 et 4, caractérisé en ce que les agents d'allongement de chaîne sont des polyols.

7. Procédé selon les revendications 3 et 4, charactérisé en ce que les agents d'allongement de chaîne sont des poly amines.

8. Polyisocyanates bloqués à base de 1 - isocyanato - 3 - isocyanatométhyl - 3,5,5 - triméthylcyclohexane et d'amidines cycliques et le cas échéant d'agents d'allongement de chaîne, obtenues selon les revendications 1—7.

**Claims**

1. A process for the preparation of blocked polyisocyanates by reaction of polyisocyanates with N-containing cyclic compounds, wherein cyclic amidines of the general formula

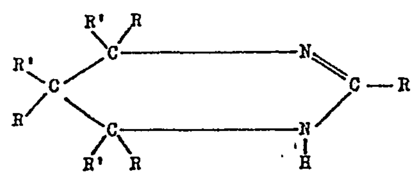

are used as N-containing cyclic compounds, wherein R are like or unlike substituents of the hydrogen-, alkyl-, cycloalkyl-, aralkyl- an aryl-group and R' R or 2 R' together are a constituent of a cycloaliphatic ring, and the reaction is carried out at temperatures of 0—150°C, preferably 80—120°C.

2. A process according to Claim 1, wherein the polyisocyanates and the cyclic amidines are used in such quantities that for one isocyanate group there is 0.5—1.1, preferably 0.8—1.0 mole cyclic amidine.

3. A process according to Claim 2, wherein the blocked polyisocyanates, for which cyclic amidines were used in substoichiometric quantities, are subsequently subjected to a reaction with chain extension agents at temperatures in the range of 0—150°C, but below the unblocking temperature, and, if necessary, the excess chain extension agent is either removed by itself or with a possibly used solvent, in a known manner.

4. A process according to Claim 3, wherein the reaction is carried out at temperatures in the range of 80—120°C.

5. A process according to Claims 3 and 4,

wherein the chain extension agent is water.

6. A process according to Claims 3 and 4, wherein the chain extension agents are polyols.

7. A process according to Claims 3 and 4, wherein the chain extension agents are polyamines.

8. Blocked polyisocyanates on basis of isophorone diisocyanate and cyclic amidines and, if necessary, chain extension agents, according to Claims 1—7.

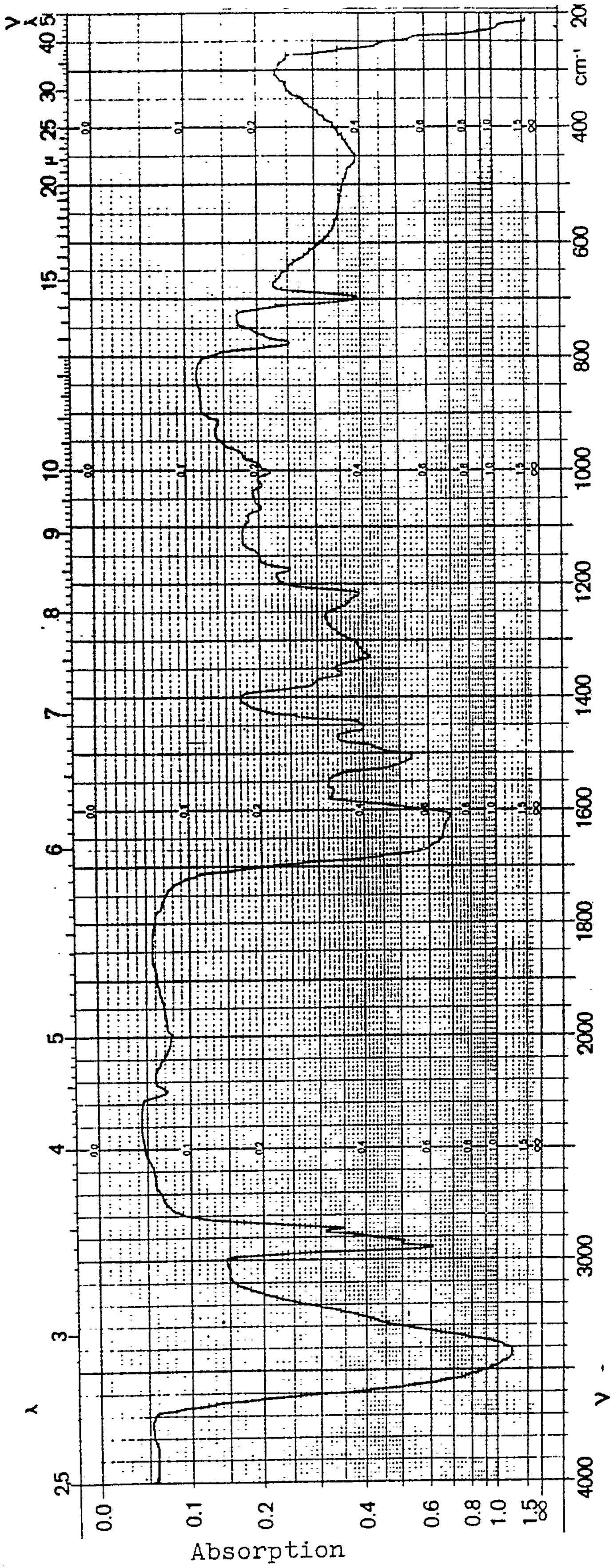
Absorption

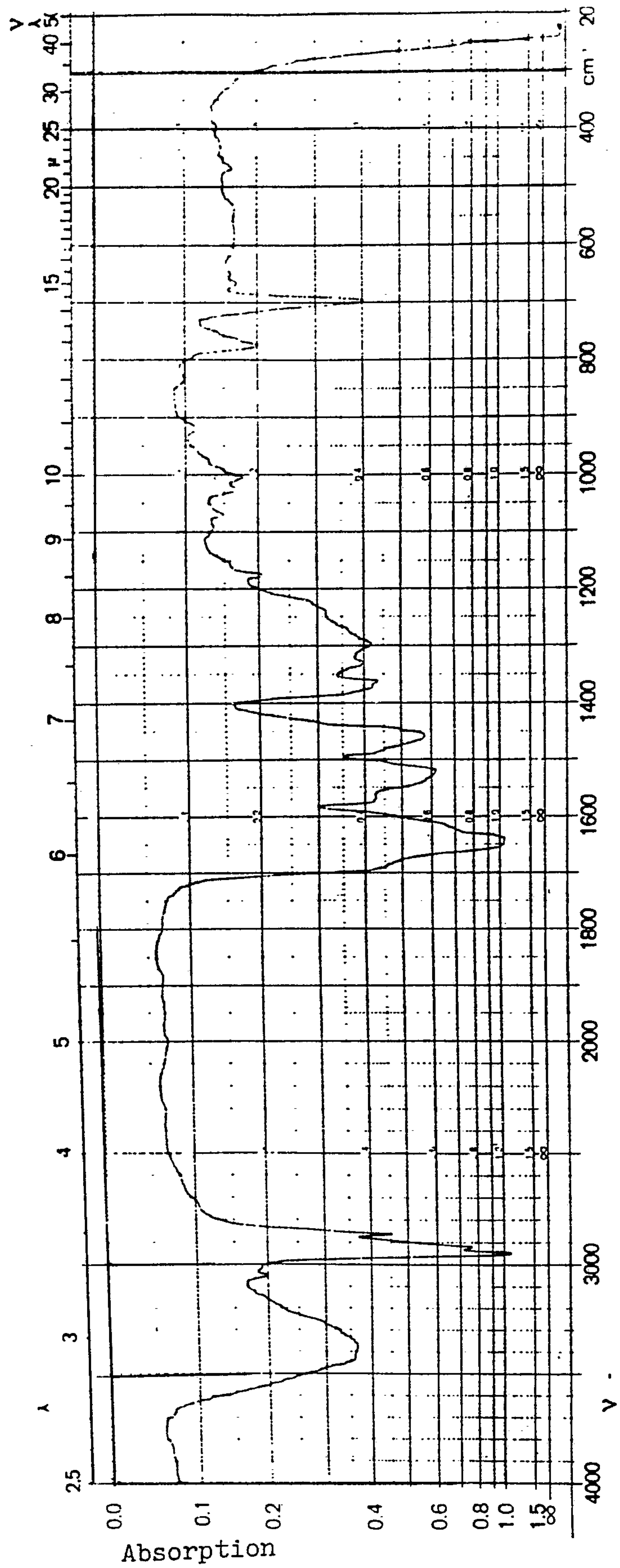
Absorption
0.0
0.1
0.2
0.4
0.6
0.8
1.0
1.5
∞
cm⁻¹
400
600
800
1000
1200
1400
1600
1800
2000
3000
4000

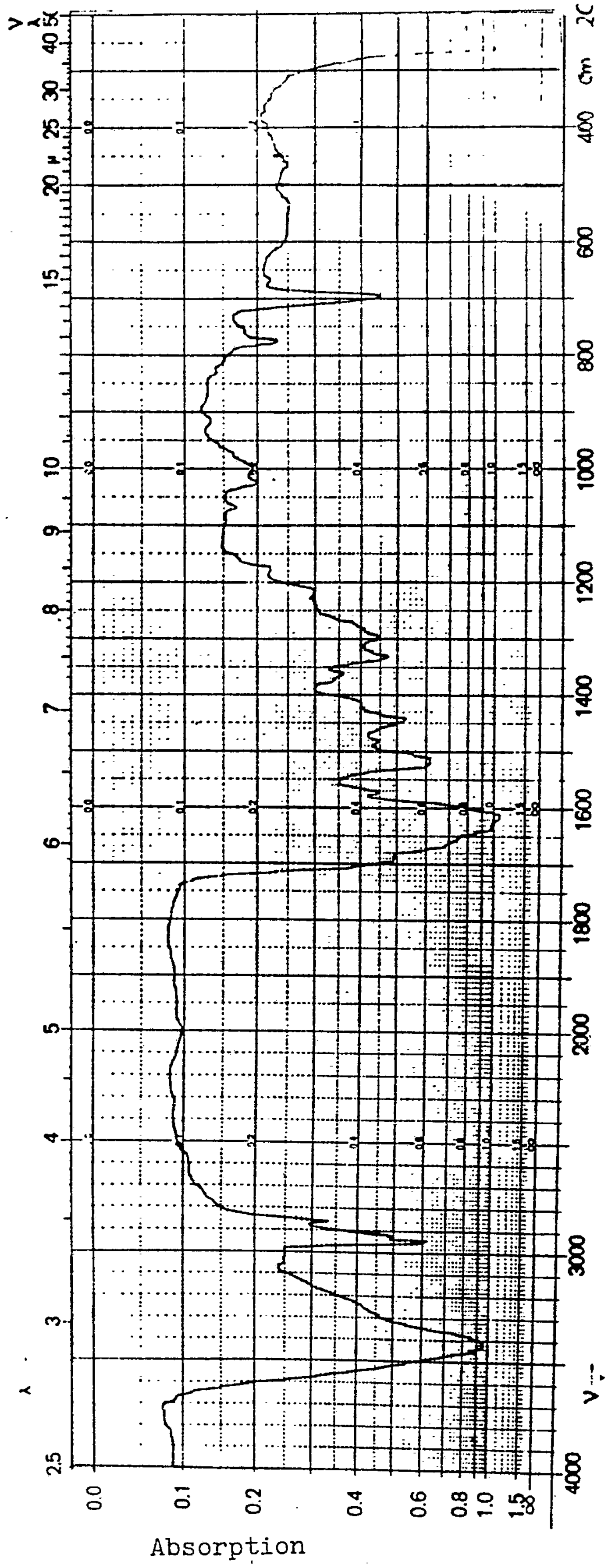

2.5
3
4
5
6
7
8
9
10
15
20 μ
25
30
40
50
4000
3000
2000
1800
1600
1400
1200
1000
800
600
400
cm
0.0
0.1
0.2
0.4
0.6
0.8
1.0
1.5
∞


Absorption

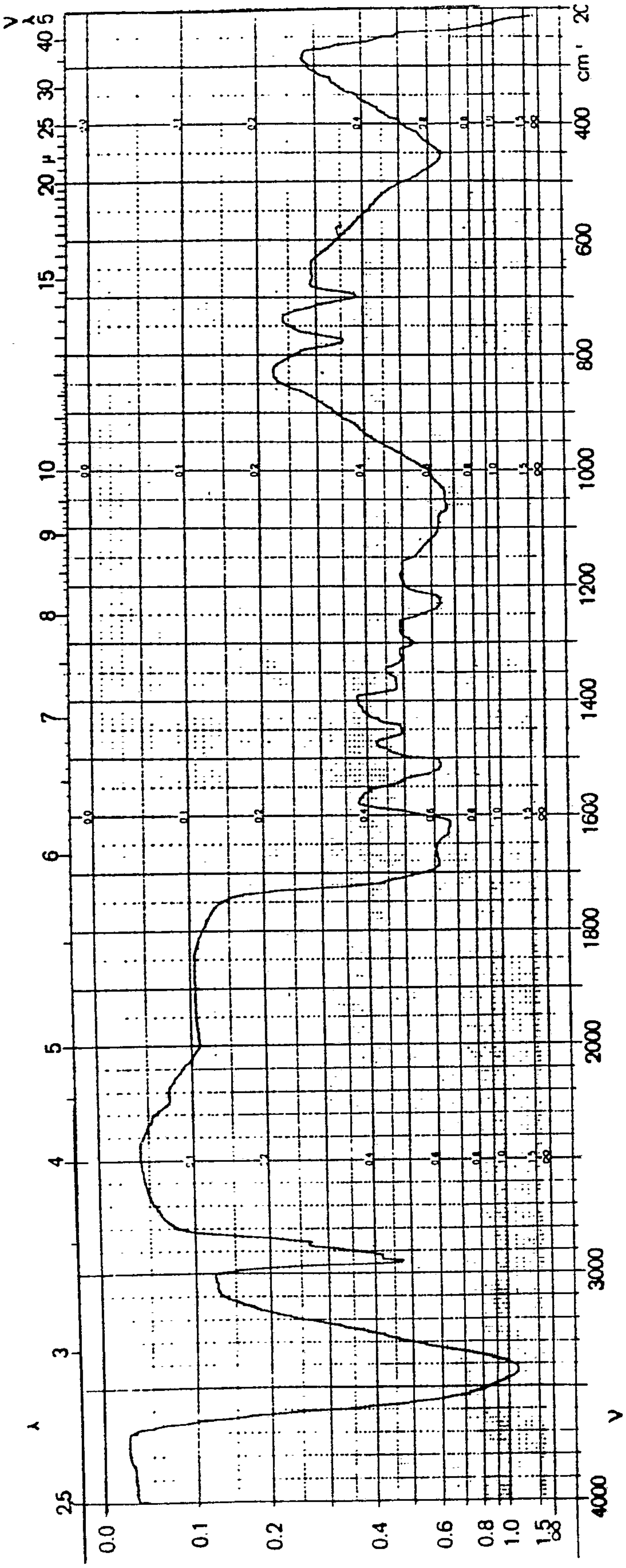
λ
ν
cm⁻¹
μ
2.5
3
4
5
6
7
8
9
10
15
20
25
30
40
4000
3000
2000
1800
1600
1400
1200
1000
800
600
400
0.0
0.1
0.2
0.4
0.6
0.8
1.0
1.5
∞

Absorption

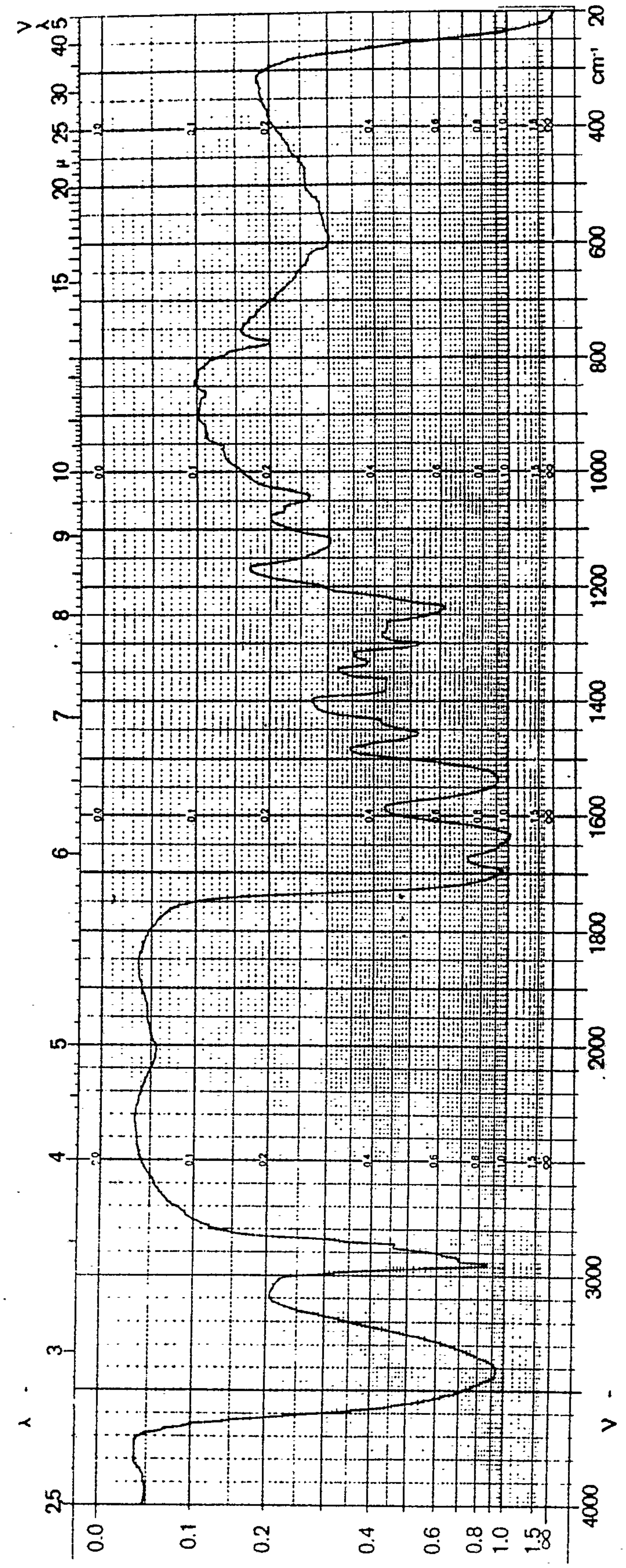

Absorption

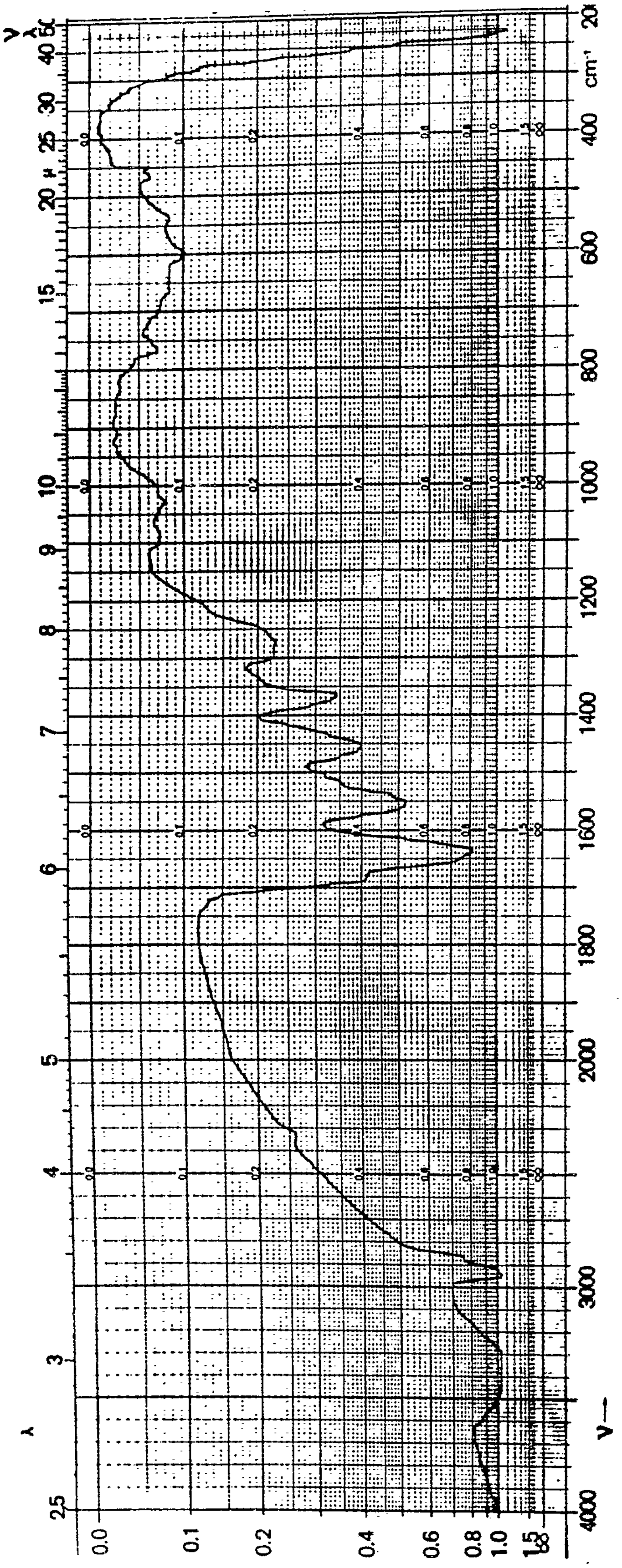

cm⁻¹
4000
3000
2000
1800
1600
1400
1200
1000
800
600
400
200
ν
λ
2.5
3
4
5
6
7
8
9
10
15
20 µ
25
30
40
50
0.0
0.1
0.2
0.4
0.6
0.8
1.0
1.5
∞


Absorption

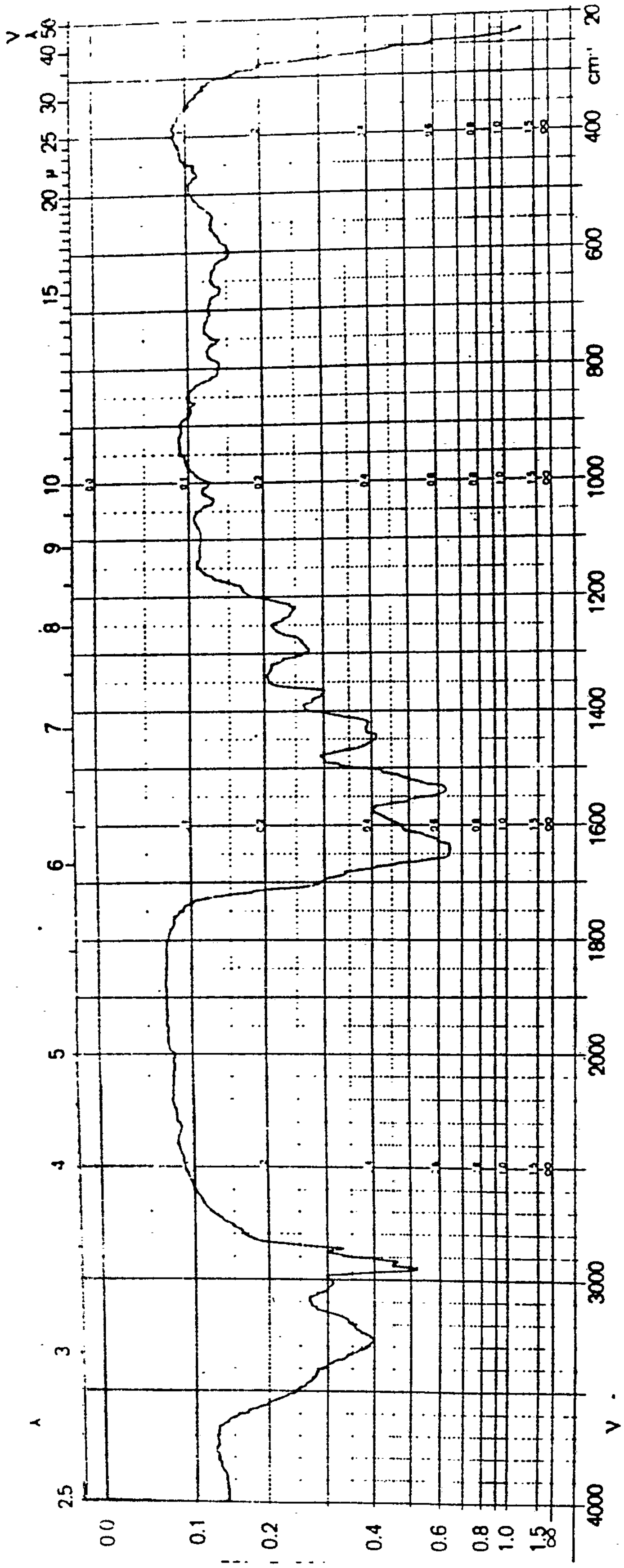

Absorption

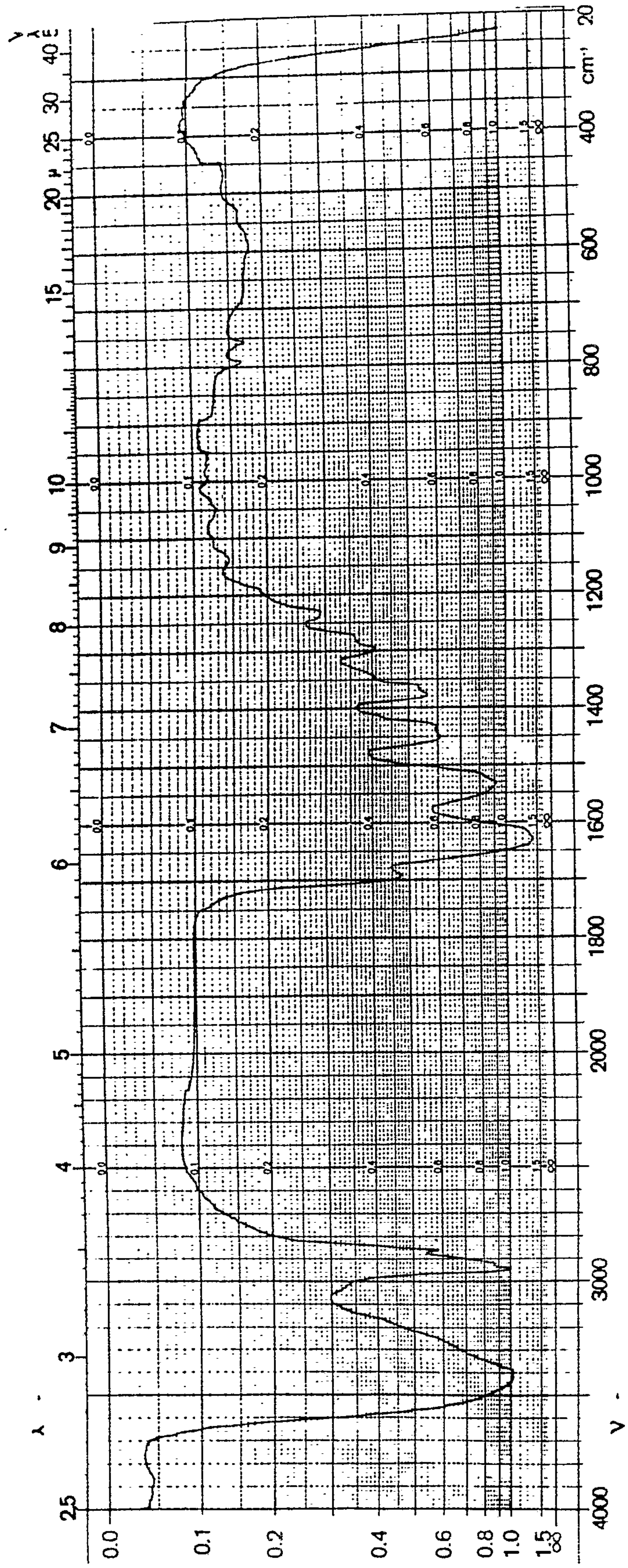

λ
2.5
3
4
5
6
7
8
9
10
15
20 μ
25
30
40
ν
4000
3000
2000
1800
1600
1400
1200
1000
800
600
400
cm⁻¹
20
0.0
0.1
0.2
0.4
0.6
0.8
1.0
1.5
∞

Absorption

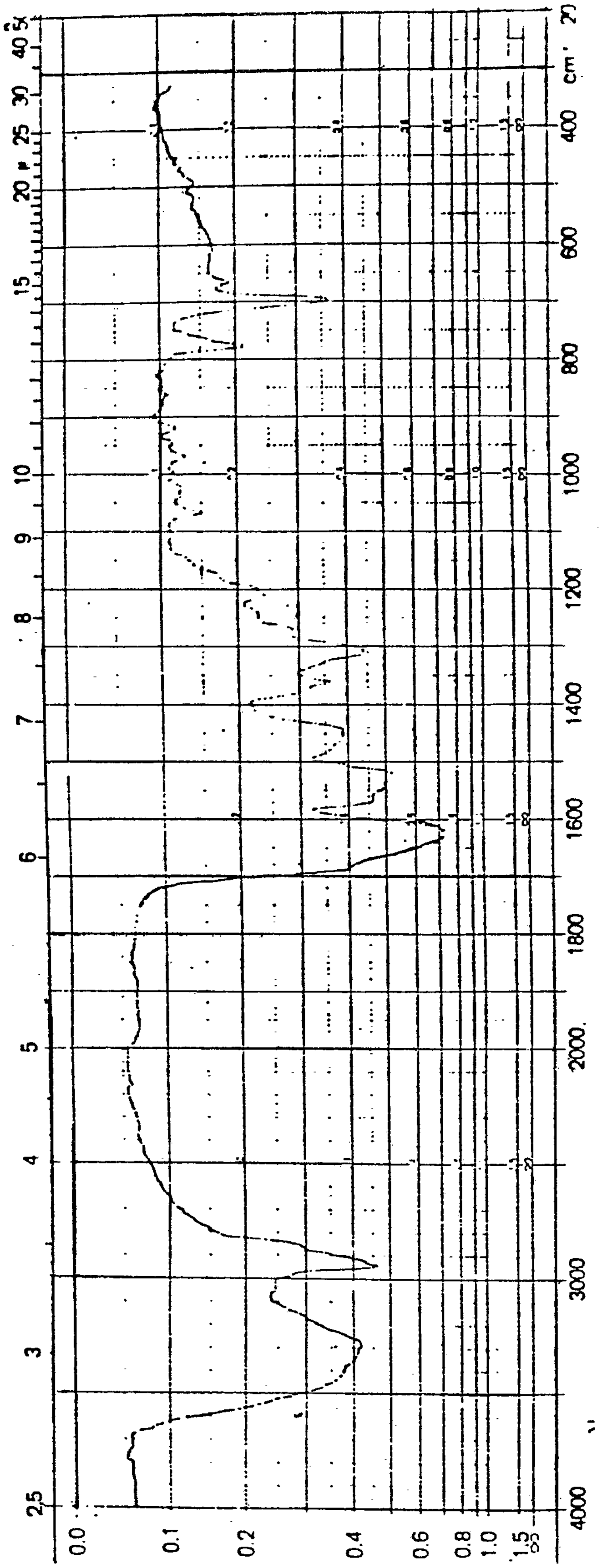

Absorption

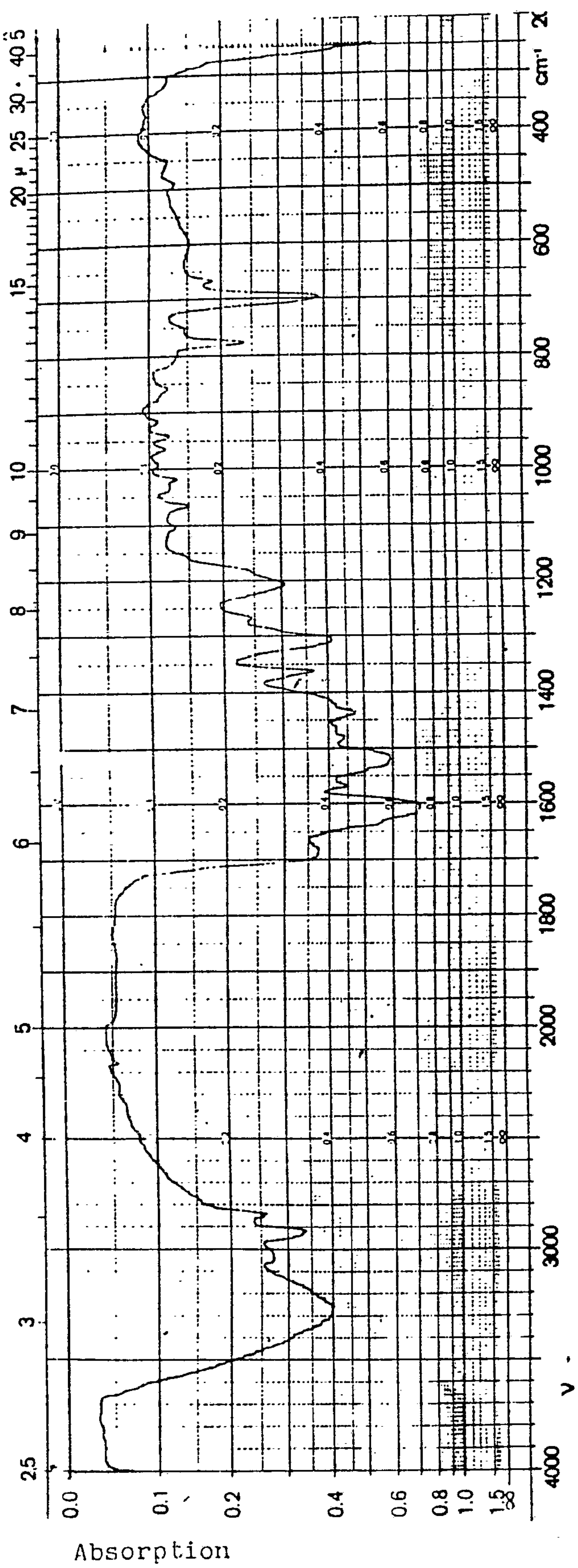
Absorption